# EUROPEAN PATENT APPLICATION

(11) **EP 2 165 735 A1**
(43) Date of publication of application: **24.03.2010**
(21) Application number: 08718424.8
(22) Date of filing: 16.01.2008
(51) Int. Cl.: A61N 2/04

(54) **BIOPHYSIOLOGICAL REGULATOR FOR THERAPEUTIC TREATMENTS**

(30) Priority: 09.07.2007 ES 200701921
(71) Applicant: Laboratorios BZ I & D, S.L., 28015 Madrid (ES)
(72) Inventor: ZAPATA PÉREZ, Carlos, E-28015 Madrid (ES)
(74) Representative: Riera Blanco, Juan Carlos
(86) International application number: PCT/ES2008/000023
(87) International publication number: WO 2009/007472

(57) **Abstract**

The invention relates to a biophysiological regulator for therapeutic treatments, including brain stimulation coils (7) which generate current using a microcontroller (11). The invention includes a second microcontroller (16), a liquid crystal display (17), a selector (21) for selecting pre-recorded programs and a connection port (20) for an external device (19) which is provided with a speaker (24) and LEDs that indicate whether or not the battery is charged (18), the regulator is connected (27), an application is underway (26) and the battery is low (25), and an internal program (28) which regulates the frequency and typology of the output wave and the time. The invention also includes a tamper protection system formed by a microcontroller (29) inside a special connector (30) built into the assembly (applicator and cable), in which the internal software of the device reads the serial number located inside the microcontroller of the connector.

## Description

### OBJECT OF THE INVENTION

The invention, as expressed in the statement of the present descriptive memory, refers to a bio-physiological regulator for therapeutic treatment.

To be more precise, the object of the invention consists of a regulating device which, associated with a series of coils subjected to electrical current, and through a micro-controller, generates a low frequency magnetic field, which provides transcraneal stimulation, being specifically designed, thanks to the internal software configuration and of the disposition of the controls it presents, to be applied as therapy in fibromyalgias and other character illnesses directly by the patients suffering from them, providing the means of regulation of the frequency as well as the output wave typology and the time of application of the same.

The regulator also incorporates a second micro-controller which monitors the battery charge level, a session counter (those already carried out, and those left), shows the user's name, etc., in a liquid display and also incorporates a series of programmes in its internal memory, which provide it with complete autonomy.

The regulator also presents a configuration in which the system's different functions are separated, in such a way so that any stage that may cause a possible malfunction can be easily located and replaced.

The device allows, in this way, to present information about the data relative to the session without the need for connection to an external device, as well as being able to choose between a series of predefined or mixed programmes.

On the other hand, the regulator to be described incorporates a key-activated switch which allows the user to incapacitate the stimulation function, independently from those of load and continuity verification.

Lastly, it must be pointed out that the proclaimed regulator, by providing its own power supply, does not need an external device, but can be complemented with one so that it may be presented with components of reduced dimensions thus allowing it a format of compact portability.

### FIELD OF APPLICATION

The field of application of the present invention is that of the industry dedicated to the manufacture of instruments and/or therapeutic devices, especially those destined for the treatment of brain-affected illnesses.

### BACKGROUND OF THE INVENTION

In therapies for the curing of illnesses of a cerebral nature, low intensity and low frequency stimulators are often used, which are essentially constituted by a magnetic field generator to which stimulation coils are connected.

With this, and the reference of the state of this technique in mind, the requester has knowledge of the P200501477 patents in relation to a type of device concerned here, which incorporates a series of coils arranged in an application cap, which supplies a current generated by a digital micro-controller powered by an external source via a tension regulator incorporating a programme recorded in its memory, in charge of generating different kinds of waves through the parameters sent by a computer to which it is connected.

In order to allow checking the correct functioning of the cable and coils, in a certificate added to said patent, an improved circuit or intermediate stage is incorporated to the start-up process which generates a warning signal when they are not in perfect working order.

Said stimulator, as well as other known devices, presents a fundamental problem in having to depend on a computer in order to introduce stimulation parameters, clearly limiting its portable possibilities, and also lacks its own screen where the information corresponding to its use or battery level may be visualised.

On the other hand, another disadvantage that said appliances present is that the different system functions are not separated in their internal configurations, which enormously complicates operations of localisation and replacing of possible mal-functioning stages.

Finally, it must be said that said devices, as the main problem to be resolved, present a lack of frequency regulation and of output wave typology of the different pre-recorded selectable programmes they incorporate.

It is, therefore, the aim of the present invention to provide an improved solution and an alternative to the problems described, and that on behalf of the applicant it should be mentioned that the existence of a similar device presenting equivalent structural technical characteristics and configuration is unknown to him.

### EXPLANATION OF THE INVENTION

For this reason, and to be precise, the bio-physiological regulator for therapeutic treatment proposes the present invention; from conventional structuring, a switch through which an electrically powered voltage regulator is connected to a converter, associated to a micro-controller, apt for connection to a computer, which, via a digital converter, changes the digitally generated signal into an analogical one so it may be conducted to a converter which transforms it into voltage, to then undergo a conversion stage to an independent power supply from the load, and connect it to the coils in a cap or helmet and generate the applied magnetic field applied on the patient. As a characteristic feature, it incorporates a connection port which allows, with the use of a second micro-controller, the introduction of the stimulation parameters: wave type, frequency, time, current, number of sessions, acoustic warning or not, amount of acoustic warnings and types of programmes, which may be simple or mixed.

This second micro-processor is associated to a liquid crystal display or similar, through which the battery level and session count can be viewed, those used by the patient as well as those still available, and shows the user's name. All these features grant the device an autonomous capacity.

The regulator also has an acoustic signal speaker, an indicator showing the end of the application, controlled by the programming software, a stage for charging the battery, which can be internally integrated, and a series of LED-type lights, which inform the users about the current working state of the generator. A LED informs users of the charge level of the battery, which is activated when charging, and switches itself off automatically once it is fully charged, or alternatively changes colour in said circumstance. A "power on" LED, remains lit when the generator is on, a "session" application LED, which blinks when a session is in progress, and a "low battery" LED.

The bio-physiological regulator, which as described, presents a compact portable format, and is applied through a treatment helmet constructed in either plastic or an adequate textile material, with an internal disposable hygienic sleeve, with a series of coils, distributed sequentially and orderly placed following an established criteria. Said coils consist of a square bracket with a rod which goes through the cap and passes through the centre hole of the coil reel, with a "click" closing system which enables the coil to be fixed within the cap.

There is also a characteristic feature which is an internal programme with the capacity to regulate frequency as well as the typology of the output wave, which is recorded within the micro-controller, powered by the device's own battery, and its functioning is governed by the corresponding programme according to an application time control, automatically stopping at the end of the application.

The regulating device of the invention, given its portability, also presents the configuration of the helmet and its manipulation, with the advantageous particularity of configuring itself as a device of direct application by the patient, thus allowing him to apply the treatment to himself, and is especially applicable in cases of fibromyalgia and other illnesses, also incorporating an anti-tampering system constituted by a micro-controller in a special connector which is incorporated into the set (applier and cable). The device's internal software reads the serial number found inside the connector's micro-controller assuring an authorised number in future applications, so they may be used by authorised personnel.

On the other hand, it must be pointed out that the use of the stimulation parameters by the regulator, through different predefined programmes, is carried out in such a way so that each one of them receives an independent group of data to individualise the type of application, that is to say, a programme with a different frequency, time and current from another. In the case of mixed programmes, a series of parameters are sent (different frequencies, times and currents) to a unique predefined programme, thus enabling the application of different frequencies, currents and variable timing.

All this is done by an external device and the micro-controller incorporated in the regulator. The device transmits the data of frequencies, currents and times used by the predefined or mixed programmes until they reach the regulator. The micro-controller receives this data and stores it in its internal EEPROM memory. Once stored there, the user has the possibility of selecting what type of application to use, whether it be a predefined programme, using only one frequency, or a mixed programme, where different times, currents and frequencies may be used for the application.

It must also be pointed out that the method for data transmission that the regulating device of the invention uses can be wireless, via Bluetooth. The regulator uses an internal mechanism which receives the signal through this means of communication. Said mechanism is a chip incorporated in the regulator's circuit, and its task is to demodulate the input signal and to transfer the data to the micro-controller's communication channels on the regulator's circuit board.

Finally, the bio-physiological regulator for therapeutic treatment presents various working states, which can be controlled by multi-positionable switches, where each one of them defines a state. The states are as follows: "off", where the whole circuit is totally disconnected from the battery and the stimulation cap; "load", where the circuit is only connected to the battery charger; "continuity", where the battery, the stimulation cap and a horn are connected, which will make a sound indicating the good working state of the circuit and coils; "application", where the battery, the cap, the circuit components, the cable circuit and the cap applicator are connected. At the end of any session, the horn beeps as often as the regulator's programme software indicates it to.

The new bio-physiological regulator for therapeutic treatment represents, therefore, an innovative structure of previously unknown constitutive and structural characteristics for this field, reasons that together with its practicality, provide it with enough grounds to obtain the solicited privilege of exclusivity.

### DESCRIPTION OF THE FIGURES

In order to complement the description that is being carried out and to help understand the characteristics of the invention better, a set of drafts are attached, as an integral part of the present descriptive memory, in which the following has been represented in an illustrious and non-limited manner:
Figure number 1.- Shows a schematic representation of the bio-physiological regulator for therapeutic treatment, object of the invention, in which the main parts and elements and the configuration and disposition of the same are indicated.

### PREFERENTIAL MANUFACTURE OF THE INVENTION

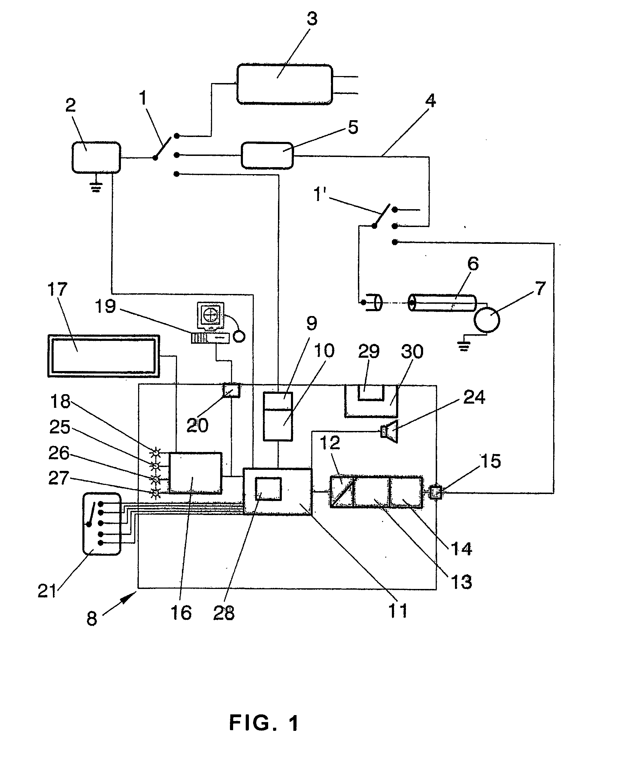

In consideration of the afore-mentioned figure 1, and according to the adopted numbering system, it can be observed that the bio-physiological regulator for therapeutic treatment comprises a double switch (1) (1'), a battery (2), a charger (3), which powers a circuit (4) with an acoustic device (5) connected to the cable (6) of the helmet with coils (7), a stimulation signal generator (8), which in turn incorporates a current regulator (9), a current converter (10) associated to a micro-controller (11) with a digital/analogue converter (12), a current/voltage converter (13) a conversion stage (14) , a special anti-tampering connector (15), also incorporating a second micro-controller (16), a liquid crystal display (17), a selector (21) of pre-recorded programmes and a connection port (20) for introducing parameters via an external device (19), a speaker (24) and a series of LED type luminous lights indicating the battery charge (18), connected regulator (27), and the application in progress (26), also incorporating an internal programme (28) which regulates the frequency as well as the wave output typology and the time of each pre-recorded programme.

When the battery voltage (2) falls below a pre-established value, a visual alarm will be produced, consisting in a blinking LED indicating a low battery (25), so that the user may perceive the situation and proceed to recharge the battery.

The re-charging stage or charger (3) of the battery (2) may be either integrated in the device, or constitute an external element, without this having an effect on the essentialness of the invention, and in either case disposing of the corresponding cable for connection to the power supply.

On the other hand, the signal generator (8) has the capacity to generate four kinds of waves: square, sinusoidal, saw-teeth and triangular, and can be limitlessly extended by carrying out a simple new transmission of data from the external device, indicating the wave-type to be used.

The selected and emitted current, through the power supply, independent from the charge, could be a coil, as in the case of experimentation at neuronal level in vitro, or various coils connected in series, as in the case of the coils cap used for the magnetic application with humans. The range of currents could surpass 2000uA.

Parameters, such as the frequency of stimulation, the current circulating through the coils (7), the time of stimulation and the wave type, are introduced through the mentioned external device (19), for which the generator (8) is provided with the corresponding connection port (20), holding enough capacity to detect whether there is an external device connected or not, in a way that should it be the first case, it will wait until the new parameters of stimulation arrive from the device (19) and will store them in the micro-controller's EEPROM (11). In the case that the system detects that no device is connected, the stimulation parameters will be read from the EEPROM memory, which will make its dependency on a device unnecessary.

On the other hand, so as to allow an acoustic signal that will indicate the end of the stimulation time to the patient or user, the device incorporates a speaker (24) controlled by the programme software, through which a beep will be emitted, repeated as often as has been programmed in order to indicate the end of the session.

Lastly, the circuit (4) is associated to a key-activated switch (not represented in the figures), which allows the user of the equipment to incapacitate the stimulation function, while the functions of charging and verification of continuity are still activated.

The device incorporates, as previously mentioned, an anti-tampering system comprising a micro-controller (29) within a special connector (30) which is incorporated into the regulator device grouping, that is to say, the application cap and cable (6), in which the device's internal software reads the serial number which is found inside the connector's micro-controller, assuring an authorised number in future applications, so they may be only used by authorised personnel.

The regulator presents a compact format of a portable nature, and is carried out through a treatment which is applied through a helmet constructed in either plastic or an adequate textile material, with an internal disposable hygienic sleeve, and with a series of coils (7), distributed sequentially and orderly placed following an established criteria. Said coils consist of a square bracket with a rod which goes through the cap and passes through the centre hole of the coil reel, with a "click" closing system which enables the coil to be fixed within the cap.

Having sufficiently described the nature of the present invention, as well as the way in which to put it into practice, it is not deemed necessary to extend the present explanation so that any expert on the subject may understand the scope and advantages that the invention presents, ascertaining that within its essentiality, it could be used in ways that differ from the details here expressed as an example, and which may equally reach the protection mentioned as long as its fundamental principle is not altered , changed or modified.

## Claims

1. The BIO-PHYSIOLOGICAL REGULATOR FOR THERAPEUTIC TREATMENT, of the type used in therapy using magnetic waves, applicable to any brain related illnesses and mainly fibromyalgias, that can be used by the own patient so that he may apply the treatment himself, in which a series of coils are employed (7) for cerebral stimulation, through which a current flows, generating a low frequency magnetic field, and is generated by a digital micro-controller (11) which is powered by a power supply, and with the means for the checking of the correct functioning of said coils (7), **characterised by** the fact that it comprises a second micro-controller (16), a liquid crystal display (17), a selector (21) of pre-recorded programmes and a connection port (20) for introducing parameters via an external device (19), a speaker (24), and a series of LED lights indicating the battery charge (18), connected regulator (27), the application in progress (26), and low battery (25) as well as an internal programme (28) which regulates the frequency as well as the wave output typology and the time of each pre-recorded programme.

2. The BIO-PHYSIOLOGICAL REGULATOR FOR THERAPEUTIC TREATMENT, according to CLAIM 1, **characterised by** the incorporation of an anti-tampering system constituted by a micro-controller (29) within a special connector (30) which is incorporated in the grouping, applicator and cable (6), in which the device's internal software reads the serial number found inside the connector's micro-controller.

3. The BIO-PHYSIOLOGICAL REGULATOR FOR THERAPEUTIC TREATMENT, according to CLAIMS 1 and 2, **characterised by** the fact that it presents a compact format of a portable nature, which is applied through a helmet constructed in an adequate textile material, with an internal disposable hygienic sleeve, and with a series of coils (7), distributed sequentially and orderly placed following an established criteria which consist of a rod which goes through the cap and passes through the centre hole of the coil reel, with a "click" closing system which enables the coil to be fixed within the cap.

## Amended claims

### Amended claims under Art. 19.1 PCT

**1.** The BIO-PHYSIOLOGICAL REGULATOR FOR THERAPEUTIC TREATMENT, of the type used in therapy using magnetic waves, applicable to any brain related illnesses and mainly fibromyalgias, that can be used by the own patient so that he may apply the treatment himself, in which a series of coils are employed (7) for cerebral stimulation, through which a current flows, generating a low frequency magnetic field, and is generated by a digital micro-controller (11) which is powered by a power supply, and with the means for the checking of the correct functioning of said coils (7), comprising a selector (21) of pre-recorded programmes, a connection port(20) for the introduction of parameters via an external device (19), **characterised by** the fact that it comprises a second micro-controller (16) a liquid crystal display (17) a speaker (24) and a series of LED lights indicating the battery charge (18), connected regulator (27), the application in progress (26), and low battery (25) as well as an internal programme (28) which regulates the frequency as well as the wave output typology and the time of each pre-recorded programme.

**2.** The BIO-PHYSIOLOGICAL REGULATOR FOR THERAPEUTIC TREATMENT, according to CLAIM 1, **characterised by** the incorporation of an anti-tampering system constituted by a micro-controller (29) within a special connector (30) which is incorporated in the grouping, applicator and cable (6), in which the device's internal software reads the serial number found inside the connector's micro-controller.

**3.** The BIO-PHYSIOLOGICAL REGULATOR FOR THERAPEUTIC TREATMENT, according to CLAIMS 1 and 2, **characterised by** the fact that it presents a compact format of a portable nature, which is applied through a helmet constructed in an adequate textile material, with an internal disposable hygienic sleeve, and with a series of coils (7), distributed sequentially and orderly placed following an established criteria which consist of a rod which goes through the cap and passes through the centre hole of the coil reel, with a "click" closing system which enables the coil to be fixed within the cap.

Statement under Art. 19.1 PCT
On the other hand, and in reference to the patents and documents seen anticipating the invention, this party would like to point out the following:

Specifically, although D1, patent WO 2004047920, describes a device which uses a memory to store different programming modes and a processor which allows an adequate selection to be made by the user, that D2, patent US 2005182287, claims another device with a micro-controller, associated memory and user interface for the selection of different programmable modes of operation, and that D3, patent GB 2413284, refers to a portable device including a coil to provide relief for depressive states, comprising an internal programmable controller which allows the user to establish the different operation modes, none of these devices can use their own means that allow the visualisation of information corresponding to their usage, nor do they allow the regulation of the frequency and output wave typology of the different programmes that may be chosen.

They are, therefore, devices that although they are destined for similar uses as the invention proposes, and are based, as the invention, on the emission of low frequency waves, present notorious differences.

To be precise, said devices also lack a second micro-controller which allows said frequency regulation and output wave typology of the programmes that may be selected from those that may be stored in their memory, do not comprise, as this one does, of a crystal display in which the battery level, session count or user name may be observed, and do not count with an anti-tampering system.
